# EUROPÄISCHE PATENTANMELDUNG

(11) **EP 1 800 621 A1**
(43) Veröffentlichungstag der Anmeldung: **27.06.2007**
(21) Anmeldenummer: 05028326.6
(22) Anmeldetag: 23.12.2005
(51) Int. Cl.: A61F 2/16

(54) **Intraokularlinse**

(71) Anmelder: Menapace, Rupert, 1190 Wien (AT)
(72) Erfinder: Menapace, Rupert, 1190 Wien (AT)
(74) Vertreter: Schmitz, Hans-Werner

(57) **Zusammenfassung**

Die Erfindung betrifft eine Intraokularlinse (1) mit einem Optikkörper (2); und einer Haptikanordnung (3, 3'), die an der Außenfläche (4) des Optikkörpers (3) befestigt ist, wobei zumindest ein Magnet (5, 5'; 6, 6'; 7, 7') am Optikkörper (2) befestigt ist.

## Beschreibung

Die Erfindung betrifft eine Intraokularlinse gemäß dem Oberbegriff des Anspruches 1.

Aus der DE 199 04 441 C1 ist ein akkommodatives Intraokularlinsensystem bekannt, das eine gattungsgemäße Intraokularlinse aufweist, die ins Innere des Kapselsackes eines Auges implantiert wird. Hierfür weist die gattungsgemäße Intraokularlinse einen Optikkörper auf, der üblicherweise aus Silikon oder Acryl besteht und an dessen Außenfläche eine sog. Haptik befestigt ist, mit der die Intraokularlinse im Kapselsack verspannt werden kann.

Die Operationstechnik, mit der das aus der DE 199 04 441 C1 bekannte Intraokularlinsensystem implantiert wird, stellt eine Technik dar, bei der das Kapsel-Kunstlinsendiaphragma in seiner Gesamtheit durch Schrumpfung und Verhärtung des Kapselsackes mehr oder weniger immobilisiert wird. Dementsprechend wird zunächst eine Art Kapselreif bzw. Spannring in den Kapselsack eingesetzt, der den entleerten Kapselsack zirkulär ausspannt und durch Verschweißen der Enden eine Schrumpfung verhindern soll. In diesen Spannring werden Magnete eingesetzt. Danach wird die zuvor beschriebene herkömmliche Intraokularlinse implantiert. Durch die Magnetwirkung zwischen den im Kapselsack angeordneten Magneten und weiteren an der Sklera angeordneten Permanentmagneten soll im Zuge der Ziliarmuskelkontraktion und dem Erschlaffen der Linsenaufhängefasern im Rahmen der Akkommodationsanstrengung das ganze, infolge der Schrumpfung plan ausgespannte Kapsel-Kunstlinsendiaphragma dosiert nach vorne geschoben werden. Deswegen sind die Magneten auch in der Peripherie verortet.

Durch diese geometrische Anordnung und eine geeignete Polarisierung der Magnete soll es möglich sein, dass sich die im Kapselsack angeordneten inneren Magnete und die an der Sklera angeordneten äußeren Magnete abstoßen und dadurch eine resultierende Kraft nach vorne erzeugen, die das Kapsel-Kunstlinsendiaphragma nach vorne verschiebt, wenn sich der Ziliarmuskel kontrahiert.

Bei dieser bekannten Technik ergeben sich jedoch zunächst insofern Probleme, als die Notwendigkeit eines zweiten Implantates in Form des speziellen Kapselspannringes das Gewicht im Auge und die Kosten des Eingriffes erhöhen. Ferner ist die Notwendigkeit einer nachträglichen Laserverschweißung aufwendig und schwierig. Des weiteren haben im Rahmen der Erfindung durchgeführte Untersuchungen ergeben, dass häufig die Schweißstellen den Schrumpfungskräften des Kapselsackes nicht widerstehen können. Somit ist das erklärte Ziel der bekannten Konstruktion, nämlich eine nachträgliche Schrumpfung der Kapsel und damit eine Anspannung der Zonularfasern im akkommodativen Ruhezustand zu verhindern, zumindest nicht immer erreichbar.

Es ist daher Aufgabe der vorliegenden Erfindung, eine Intraokularlinse der im Oberbegriff des Anspruches 1 angegebenen Art zu schaffen, die es möglich macht, im implantierten Zustand eine zufriedenstellende und dauerhafte Akkommodationswirkung bei sich kontrahierendem Ziliarmuskel zu erreichen.

Die Lösung dieser Aufgabe erfolgt durch die Merkmale des Anspruchs 1.

Dadurch, dass die inneren Magnete im Gegensatz zu dem zuvor beschriebenen Intraokularlinsensystem nicht in der Peripherie des Kapselsackes, sondern am Optikkörper der Intraokularlinse selber befestigt sind, ergibt sich die Möglichkeit, dass nicht das Diaphragma als gesamtes, sondern nur die Optik der Intraokularlinse durch Magnetwirkung nach vorne verschoben werden kann. Es wird also nicht, wie bei der DE 199 04 441 C1, das trampolinartig ausgespannte Diaphragma bzw. der Kapselsack mit der darin befindlichen gesamten Kunstlinse parallel verschoben, sondern nur der Optikkörper der Intraokularlinse, wobei die Anbindungsstellen der Haptiken an die Optik wie Scharniere oder Sollknickstellen wirken und das Kapseldiaphragma außerhalb der Optik und die zonulare Aufhängung leicht zeltförmig angehoben und gedehnt werden. Dies ist erfindungsgemäß deshalb möglich, da die Verwendung der erfindungsgemäßen Intraokularlinse mit integrierten Magneten eine Operationstechnik ermöglicht, die eine Schrumpfung und Verhärtung des Kapselsackes zumindest weitgehend, wenn nicht gar völlig unterbindet und die Kapselelastizität erhält. Damit wird auch einer schrumpfungsbedingten unphysiologischen Anspannung der Zonularfasern im akkommodativen Ruhezustand entgegengewirkt.

Die Unteransprüche haben vorteilhafte Weiterbildungen der Erfindung zum Inhalt.

So ist es grundsätzlich möglich, einen, vorzugsweise jedoch zwei, einander gegenüber angeordnete Magnete vorzusehen, die entweder peripher liegend in den Optikkörper eingelassen sind, an dessen Außenoberfläche angebracht sind oder in einem Übergangsbereich peripher angeordnet sind, wozu es beispielsweise möglich ist, Ausnehmungen in den Optikkörper einzulassen, in den die vorzugsweise als Permanentmagnete ausgebildeten Magnete eingelassen werden können.

Bevorzugterweise sind die Magnete hierbei so ausgebildet, dass eine Neutralisierung des Gewichtes der Magneten und des Optikkörpers möglich ist, indem sie beispielsweise in Auftriebskörper integriert sind.

Ferner ist es vorzugsweise möglich, eine Einrichtung zur Zentrierung des Optikkörpers auf die Achse des Auges bzw. die Sehachse vorzusehen.

Hierzu ist vorzugsweise eine Zähnelung vorgesehen, die an der Haptik, insbesondere dem Achselbereich der Haptikanbindung, angeordnet ist, mittels der die Haptik am Optikkörper fixiert ist. Damit ist es möglich, dass die Optik auf die Augen- bzw. Sehachse einzentriert bzw. einjustiert und in dieser Position stabil fixiert gehalten werden kann. Bei einer besonders bevorzugten Ausführungsform ist zur Fassung einer Zentriereinrichtung die Linse im Bereich des Haptikansatzes durch eine Zähnelung modifiziert, die im Bereich der Achsel des Haptikansatzes angeordnet ist, eine entsprechende Neigung (Hinterschneidung) aufweist um den Kapselrand sicher zu halten und die im Bereich der Schulter des Haptikansatzes einen gleichmäßig verlaufenden Übergang vom Rand des Linsenkörpers zur konvex gekrümmt verlaufenden bügelförmigen Haptik aufweist, um eine Verschiebung der Überkreuzungsstelle zwischen Kapsel- und Linsenrand in Richtung Achsel durch Aufgleiten der Überkreuzung auf den konkav gestalteten Abgang der bügelförmigen Haptik zu ermöglichen. Hierdurch kann eine Verschiebung des Haptikansatzes und damit eine Rezentrierung der Optik in Bezug auf die Achse des Kapselsackes bei primär dezentrierter Optik im Rahmen der nachstehend beschriebenen Operationstechnik erreicht werden.

Eine derartige Zentriereinrichtung an der Haptik kann hierbei sowohl bei einer erfindungsgemäßen Intraokularlinse mit Magneten als auch bei herkömmlichen Intraokularlinsen ohne Magneten vorgesehen sein und stellt somit eine selbständig handelbare Einheit im Zusammenhang mit der Haptikanbindung einer implantierbaren Intraokularlinse dar.

Eine derartige Zentriereinrichtung wird vorzugsweise bei Intraokularlinsen verwendet, die mit einer Operationstechnik implantiert werden, bei der in der vorderen und hinteren Linsenkapsel eine möglichst kreisförmige und mittig angeordnete Öffnung mit kontinuierlichem Rand (sogenannte Kapsulorhexisöffnung) angebracht wird. Die Intraokularlinse wird dabei in der Weise im Auge fixiert, dass sie zunächst in ihrer Gesamtheit in den Kapselsack eingebracht und die beiden Haptiken zwischen den beiden Kapselblättern im so genannten Kapselsackfornix positioniert werden, während die Optik sekundär ähnlich dem Hindurchstecken eines Knopfes durch ein Knopfloch durch die hintere Kapsulorhexisöffnung nach hinten eingeknöpft wird. Alternativ ist auch ein Einknöpfen der Optik durch die vordere Kapsulorhexisöffnung nach vorne möglich. Als weitere Alternativen können die beiden Haptiken vor dem vorderen Kapselblatt im so genannten Sulkus ciliaris fixiert und die Optik sekundär durch die vordere oder die vordere und hintere Kapsularhexisöffnung nach hinten durchgeknöpft werden.

Die zusätzliche Zentriereinrichtung unterstützt die Selbstzentrierung durch die Linsenbügel nun dadurch, dass bei der Verwendung einer Zähnelung im Achselbereich der Haptikanbindung durch Einhaken des Randes der hinteren Ausnehmung des Kapselsackes und entsprechende Wahl der Verzahnung es dem Operateur im Falle einer dezentrierten hinteren Kapselausnehmung möglich gemacht wird, dennoch die Achse der Intraokularlinse auf die Achse des Auges oder des Sehens einzuzentrieren.

Weitere Einzelheiten, Vorteile und Merkmale der vorliegenden Erfindung ergeben sich aus nachfolgender Beschreibung von Ausführungsbeispielen anhand der Zeichnung. Es zeigt:
- Fig. 1: eine schematische stark vereinfachte Darstellung einer Intraokularlinse gemäß der vorliegenden Erfindung,
- Fig. 2A: eine der Fig. 1 entsprechende vereinfachte Darstellung einer implantierten erfindungsgemäßen Intraokularlinse, entlang der Schnittlinie I-I in Fig.1,
- Fig. 2B: eine der Fig. 1 entsprechende vereinfachte Darstellung einer implantierten erfindungsgemäßen Intraokularlinse entlang der Schnittlinie II-II in Fig. 1,
- Fig. 3A: eine der Fig. 1 entsprechende Darstellung der erfindungsgemäßen Intraokularlinse,
- Fig. 3B und 3C: Detailansichten weiterer Ausführungsformen der in Fig. 1 und 3A dargestellten Intraokularlinse, und
- Fig. 4: eine der Fig. 1 entsprechende Darstellung der erfindungsgemäßen Intraokularlinse zur Erläuterung einer besonders bevorzugten Ausführungsform einer erfindungsgemäßen Zentriereinrichtung.

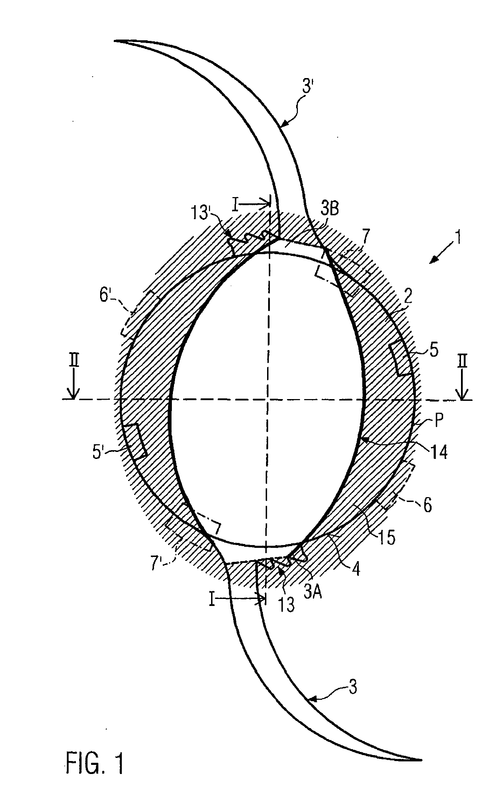

Gemäß Fig. 1 weist die erfindungsgemäße Intraokularlinse 1 einen üblicherweise linsenförmig ausgeführten Optikkörper 2 aus optischem Material, insbesondere faltbarem Silikon- oder Acryl auf.

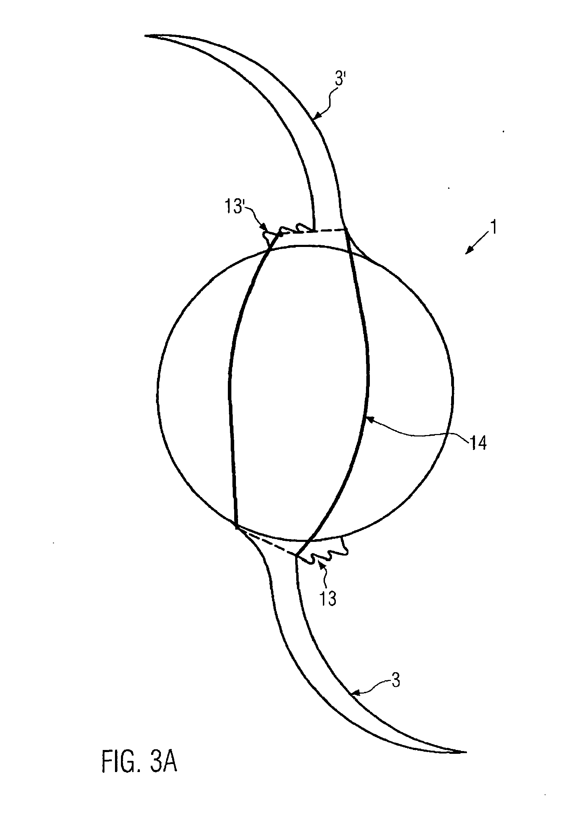

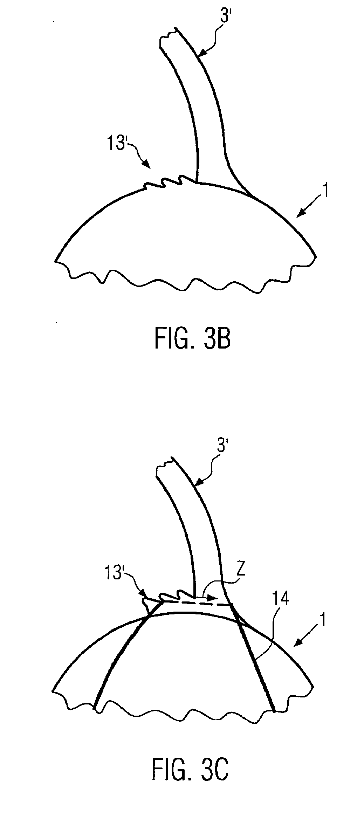

Im Beispielsfalle ist an der Außenfläche 4 des Optikkörpers 2 eine Anordnung aus zwei Haptiken 3, 3' befestigt. Diese Haptiken 3, 3' sind bügelförmig ausgebildet und weisen eine Haptikbasis 3A bzw. 3B auf, die im Beispielsfalle jeweils mit einer Zentriereinrichtung 13 bzw. 13' versehen ist. Wie Fig. 1 verdeutlicht, sind die Zentriereinrichtungen 13, 13' jeweils als Zähnelung ausgebildet, die in der zuvor beschriebenen Art und Weise mit dem Rand 14 einer hinteren Kapselausnehmung der hinteren Linsenkapsel 15 (schraffierte Fläche) im Kapselsack zusammenwirken, was nachfolgend anhand der Fig. 3A-3C ergänzend erläutert werden wird.

Wie Fig. 1 ferner verdeutlicht, weist der Optikkörper 2 im Bereich seiner Peripherie P eine Magnetanordnung auf. Hierbei sind in Fig. 1 drei alternative Möglichkeiten gleichzeitig dargestellt, von denen in der Praxis vorzugsweise nur eine Verwendung findet.

Die erste Magnetanordnung weist zwei gegenüber angeordnete Magnete 5 und 5' auf, die im Inneren des Optikkörpers 2 an der Peripherie P angeordnet sind.

Die zweite Magnetanordnung weist zwei gegenüber angeordnete Magnete 6 und 6' auf, die gestrichelt wiedergegeben sind und die außen auf der Außenfläche 4 befestigt sind.

Eine dritte Alternative stellt die Magnetordnung dar, die strichpunktiert dargestellte Magnete 7 und 7' aufweist, die in einem Übergangsbereich der Außenfläche bzw. der Peripherie P angeordnet sind und somit teilweise innerhalb des Optikkörpers 2 liegen und teilweise über die Außenfläche 4 vorragen, wie sich dies im Einzelnen aus Fig. 1 ergibt.

Wie zuvor bereits betont, wird üblicherweise nur eine der drei in Fig. 1 gleichzeitig dargestellten Alternativen der Magnetanordnungen verwendet, obwohl es grundsätzlich denkbar ist, auch beliebige Kombinationen vorzusehen, also beispielsweise eine Kombination eines in den Optikkörper eingelassenen und eines auf der Außenoberfläche angeordneten Magneten.

Ferner ist es vom Prinzip her möglich, lediglich einen Magneten vorzusehen, wobei jedoch eine bevorzugte Anordnung ein Magnetpaar aufweist, die jeweils diametral gegenüberliegend angeordnet sind, wie sich dies aus Fig. 1 ergibt. Ferner ist beispielsweise auch eine dünne ringförmige magnetische Folie denkbar, die auf die Optikperipherie aufgebracht werden kann.

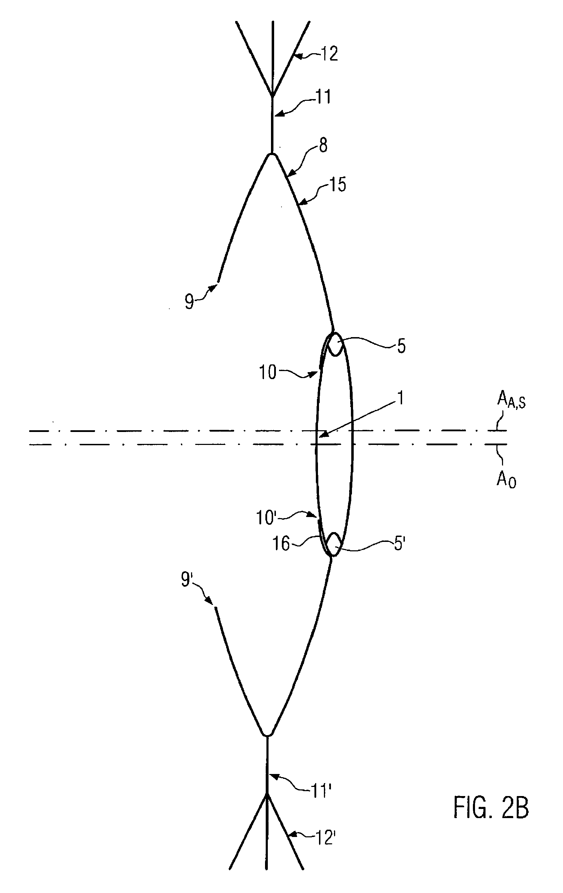

Diese Magnete, die vorzugsweise als Permanentmagnete ausgebildet sind, wirken mit (nicht dargestellten) äußeren an der Sklera des Auges angeordneten weiteren Magneten zusammen, wobei die geometrische Anordnung und Polarisierung so gewählt ist, dass eine Verschiebung der implantierten Intraokularlinse 1 möglich ist, wenn sich der Ziliarmuskel kontrahiert.

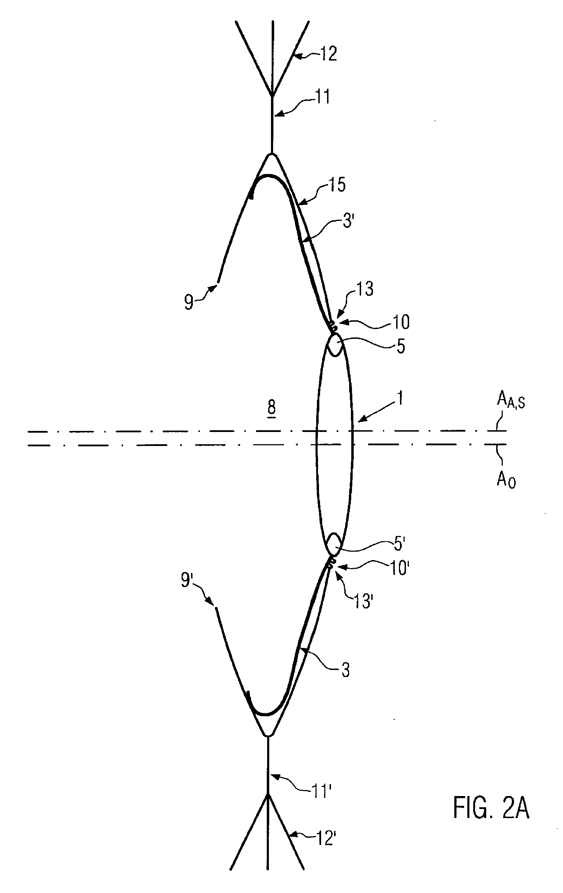

Fig. 2A verdeutlicht den implantierten Zustand der erfindungsgemäßen Intraokularlinse 1, der durch folgende Operationstechnik erreichbar ist:
   Zunächst wird im Bereich des Außenumfanges des Auges ein Schnitt angebracht, durch den der getrübte Linsenkörper dem Kapselsack entnommen werden kann, was in üblicher Operationstechnik, beispielsweise durch eine Absaugung nach Ultraschallverflüssigung, durchgeführt werden kann.
   Danach werden zwei Ausnehmungen (Kapsulorhexisöffnungen) in der vorderen und hinteren Kapsel angebracht, deren Ränder in Fig. 2 mit 9 und 9' bzw. 10 und 10' bezeichnet sind. Die vordere Ausnehmung 9/9'stellt die der Vorderkammer zugewandte Ausnehmung dar, während die hintere Ausnehmung 10/10' die dem Glaskörperraum des Auges zugewandte Ausnehmung darstellt.
   Wie Fig. 2A verdeutlicht, wird zur Implantierung die Intraokularlinse 1 zunächst in den Kapselsack eingesetzt und danach die Linsenptik durch die hintere Ausnehmung 10/10' hindurchgefädelt, ähnlich dem Einfädeln eines Knopfes in ein Knopfloch, so dass sich die Haptiken 3 und 3' innerhalb des Kapselsacks 8 verspannen und der Optikkörper 2 hinter die hintere Ausnehmung 10/10' platziert ist.
Fig.2B zeigt die implantierte Intraokularlinse 1 entlang der Schnittlinie II-II in Fig.1. Aus dieser Darstellung wird insbesondere Überlappungsbereich 16 des Randes der hinteren Kapselausnehmung 15 deutlich, der sich der Vorderfläche der Optik der Intraokularlinse 1 anlegt.

Die zuvor erwähnten Zähnelungen 13 und 13' können hierbei eine weitere Zentrierhilfe darstellen, um einen eventuellen in Fig. 2B verdeutlichten Versatz zwischen der Achse Aₒ des Optikkörpers 2 und der Augenachse bzw. Sehachse A_{AS} zu minimieren bzw. völlig auszugleichen. Eine derartige zusätzliche Zentriereinrichtung ist zwar nicht unbedingt erforderlich, da im Wesentlichen eine Zentrierung bereits durch die Haptiken 3 und 3' möglich ist, jedoch ist die zusätzliche Zentriereinrichtung 13 bzw. 13' für eine weitere Perfektionierung hilfreich.

Nach der Implantierung der erfindungsgemäßen Intraokularlinse 1 ist durch die zuvor erläuterte Magnetwirkung zwischen den am Optikkörper 2 angebrachten Magneten, wie beispielsweise den Magneten 5 und 5' der Darstellung der Fig. 2, und den in der Zeichnung nicht näher dargestellten Permanentmagneten an der Sklera des Auges eine Akkommodation durch den sich kontrahierenden ringförmigen Ziliarmuskeln 12 und 12' des Auges erreichbar, indem die Intraokularlinse 1 in Richtung der Augen- bzw. der Sehachse A_{A},ₛ nach vorne, also in Richtung Hornhaut verschoben wird.

Aus den Figuren 3A und 3C wird deutlich, dass die Zähnelung der Zentriereinrichtung vorzugsweise derart ausgestaltet ist, dass der eingezahnte Kapselrand - da unter Zug stehend - fixiert bleibt, was durch den Pfeil Z in Fig. 3C verdeutlicht ist. Vorzugsweise ist hierfür die Zähnelung 13 bzw. 13' etwas geneigt ausgebildet, was sich aus der Darstellung der Fig. 3C erschließt.

Aus den Figuren 3A-3C wird in Ergänzung zu den voranstehenden Erläuterungen deutlich, dass die Zähnelung 13' alternativ auch in den Optikkörper eingebracht, insbesondere eingefräst werden kann.

Aus der Darstellung der Fig. 3C wird ferner deutlich, dass im Zuge der axialen Verschiebung im Bereich des Haptikansatz eine scharnierartige Bewegung stattfinden kann. Das Ausmaß der Beweglichkeit kann vorzugsweise durch die Ausführung (Design, Material) des Haptikansatzes beeinflusst werden.

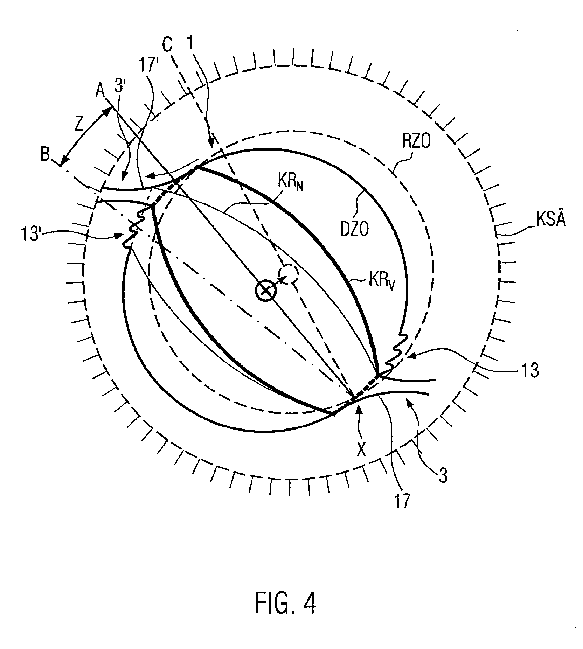

Fig. 4 verdeutlicht eine besonders bevorzugte Ausführungsform von Zentriereinrichtungen 13 und 13' der erfindungsgemäßen Intraokularlinse 1.

Hierbei markiert die durchgehende Linie DZO den Rand der primär dezentrierten Optik in der primär dezentrierten hinteren Kapselöffnung. Die strichlierte Linie RZO markiert den Rand der rezentrierten Optik nach einem entsprechenden Einhängen der einen Kapselüberkreuzung in die Zähnelung 13' und dem konsekutiven Aufgleiten der zweiten Überkreuzung auf den verlaufend ausgeführten Übergang zwischen Linsenrand und Bügelkonvexität 17'. Die resultierende typische Verziehung des Randes der hinteren Kapselöffnung ist durch den Verlauf dargestellt, der mit der Bezugsziffer KRn gekennzeichnet ist (Verlauf Rand der hinteren Kapselsacköffnung nach Justierung), während die Linie KRv den Rand der hinteren Kapselsacköffnung vor der Justierung darstellt. Die äußere gestrichelte Kreislinie KSÄ zeichnet den Kapselsackäquator, von dem die als radiale Striche symbolisierten Zellularfasern abgehen. Nach der Rezentrierung ist die Optik in Bezug auf den Kapselsack und damit auch die Achse des Auges 0 einzentriert, was beides in Fig. 4 durch die strichlierte Darstellung der Optikaussenfläche gezeigt ist. Die Verschiebung wird durch den Pfeil zwischen dem Mittelpunkt der primär dezentrierten Optik in einer primär dezentrierten hinteren Kapselöffnung einerseits und dem Mittelpunkt der Optik nach Rezentrierung anderseits (jeweils durch einen durchgezogen bwz. strichliert gezeichneten kleinen Kreis symbolisiert, wobei letzterer nunmehr der Achse des Auges bzw. des Sehens entspricht).

### Bezugszeichenliste

- 1: Intraokularlinse
- 2: Optikkörper
- 3, 3': Haptiken
- 3A, 3B: Haptik-Basis
- 4: Außenfläche
- 5, 5': Magnete, insbesondere Permanentmagnete
- 6, 6': Magnete, insbesondere Permanentmagnete
- 7, 7': Magnete, insbesondere Permanentmagnete
- 8: Kapselsack
- 9, 9': Rand der vorderen Ausnehmung des Kapselsacks 8
- 10, 10': Rand der hinteren Ausnehmung des Kapselsacks 8
- 11, 11': Zonularfaserapparat
- 12, 12': Ziliarmuskel (peripherer Ansatz der Zonularfasern)
- 13, 13': Zentriereinrichtung (Zähnelung)
- 14: Rand der hinteren Kapselausnehmung
- 15: hintere Linsenkapsel (schraffierte Fläche in Fig.1)
- 16: Überlappung
- 17, 17': Übergangsbereich Optikrand-Bügelkonvexität
- P: Peripherie
- Aₒ: Achse des Optikkörpers 2
- A_{A,S}: Augenachse bzw. Sehachse
- Z: Zugrichtung
- KRv: Verlauf des Hinterkapselrandes vor Zentrierung
- KRn: Verlauf des Hinterkapselrandes nach Zentrierung
- DZO: dezentrierte Optik
- RZO: rezentrierte Optik
- KSÄ: Kapselsackäquator

## Patentansprüche

1. Intraokularlinse (1)
- mit einem Optikkörper (2); und
- mit einer Haptikanordnung (3, 3'), die an der
Außenfläche (4) des Optikkörpers (3) befestigt ist, **dadurch gekennzeichnet,**
- **dass** zumindest ein Magnet (5, 5'; 6, 6'; 7, 7') am Optikkörper (2) befestigt ist.

2. Intraokularlinse nach Anspruch 1, **dadurch gekennzeichnet, dass** der Magnet (6, 6') außen an der Peripherie (P) des Optikkörpers (2) angeordnet ist.

3. Intraokularlinse nach Anspruch 1, **dadurch gekennzeichnet, dass** der Magnet (5, 5') im Inneren des Optikkörpers (2) an dessen Peripherie (P) angeordnet ist.

4. Intraokularlinse nach Anspruch 1, **dadurch gekennzeichnet, dass** der Magnet (7, 7') in einem Übergangsbereich der Außenfläche (4) des Optikkörpers (2) angeordnet ist.

5. Intraokularlinse nach einem der Ansprüche 1 bis 4, **dadurch gekennzeichnet, dass** eine Magnetanordnung, bestehend aus zwei diametral zueinander gegenüberliegend am Optikkörper (2) angeordneten Magneten (5, 5'; 6, 6'; 7, 7'), vorgesehen ist.

6. Intraokularlinse nach einem der Ansprüche 1 bis 5, **dadurch gekennzeichnet, dass** die Magnete (5, 5'; 6, 6'; 7, 7') so ausgebildet sind, dass ihr Gewicht und das Gewicht des Optikkörpers (2) neutralisierbar ist.

7. Intraokularlinse nach einem der Ansprüche 1 bis 6 **gekennzeichnet durch** eine Einrichtung zum Zentrieren des Optikkörpers (2) auf die Achse (A_{A,S}) des Auges bzw. des Sehens.

8. Intraokularlinse nach Anspruch 7, **dadurch gekennzeichnet, dass** die Zentriereinrichtung (13, 13') als kammartige, vorzugsweise hinterschnittene Zähnelung ausgebildet ist.

9. Intraokularlinse nach Anspruch 7 oder 8, **dadurch gekennzeichnet, dass** die Zentriereinrichtung (13, 13') an der Haptik (3, 3'), vorzugsweise der Haptik-Basis (3A, 3B) bzw. dem Achselbereich der Haptikanbindung mit einem gleichmäßig gekrümmt verlaufenden Übergangsbereich im Schulterbereich (17, 17') vom Rand des Optikkörpers (2) zur Haptik (3, 3') vorgesehen ist.

10. Intraokularlinse nach Anspruch 7 oder 8, **dadurch gekennzeichnet, dass** die Zentriereinrichtung (13, 13') am Optikkörper (2) vorgesehen ist.
